# EUROPÄISCHE PATENTANMELDUNG

(11) **EP 4 696 188 A1**
(43) Veröffentlichungstag der Anmeldung: **18.02.2026**
(21) Anmeldenummer: 24194490.9
(22) Anmeldetag: 14.08.2024
(51) Int. Cl.: A45D 29/00, A61B 17/54, B24B 23/02

(54) **HANDGERÄT ZUM ABRIEB VON HORNHAUT**

(71) Anmelder: NWT Management GmbH, 04425 Taucha (DE)
(72) Erfinder: BAUCH, Dieter, 76751 Jockgrim (DE); LAMOUREUX, Bruno, 1180 Brussels (BE)
(74) Vertreter: Maikowski & Ninnemann Patentanwälte Partnerschaft mbB

(57) **Zusammenfassung**

Die Erfindung betrifft ein Handgerät zum Abrieb von Hornhaut aufweisend ein Gehäuse, einen Rotor und einen Stator. Im Gehäuse des Handgerätes ist ein Motor mit einer Antriebswelle angeordnet. Weiterhin weist der Rotor zwei Klingen auf und kann auf die Antriebswelle des Motors aufgesteckt werden. Erfindungsgemäß wird der Rotor auf die Antriebswelle des Gehäuses gesteckt und der Stator auf den Rotor. Der Stator wird dann am Gehäuse befestigt. Vorteilhafterweise ist der Rotor des Handgerätes auswechselbar. Weiterhin umfasst die Erfindung einen Rotor zum Einsatz in einem Handgerät zum Abrieb von Hornhaut. Der Rotor weist einen zylinderförmigen Körper und zwei Klingen auf. Der zylinderförmige Köper weist eine axiale Bohrung zur Aufnahme einer Antriebswelle auf und weiterhin zwei Aufnahmevorrichtungen, wobei jede Aufnahmevorrichtung eine Klinge aufnehmen kann.

## Beschreibung

Die Erfindung betrifft ein Handgerät zum Abrieb von Hornhaut aufweisend ein Gehäuse, einen Rotor und einen Stator. Im Gehäuse des Handgerätes ist ein Motor mit einer Antriebswelle angeordnet. Weiterhin weist der Rotor zwei Klingen auf und kann auf die Antriebswelle des Motors aufgesteckt werden. Erfindungsgemäß wird der Rotor auf die Antriebswelle des Gehäuses gesteckt und der Stator auf den Rotor. Der Stator wird dann am Gehäuse befestigt. Vorteilhafterweise ist der Rotor des Handgerätes auswechselbar. Weiterhin umfasst die Erfindung einen Rotor zum Einsatz in einem Handgerät zum Abrieb von Hornhaut. Der Rotor weist einen zylinderförmigen Körper und zwei Klingen auf. Der zylinderförmige Köper weist eine axiale Bohrung zur Aufnahme einer Antriebswelle auf und weiterhin zwei Aufnahmevorrichtungen, wobei jede Aufnahmevorrichtung eine Klinge aufnehmen kann.

Aus dem Stand der Technik sind Verfahren zum Entfernen von Hornhaut bekannt, bei denen Hornhautschichten mit herkömmlichen Skalpellen oder mit einem Handgerät mit einer feststehenden Klinge abgeschabt werden. Die Gefahr von Verletzungen und Schnitten ist bei diesen Verfahren jedoch sehr groß.

Weiterhin bekannt ist ein Verfahren aus der FR 2 765 471 A1, bei dem ein Handinstrument zum Einsatz kommt, bei dem die Klingen nicht feststehend sind, sondern durch einen externen Motor rotiert werden. Das Handgerät weist einen Rotor auf, an den durch Schraubverbindungen zwei Klingen angebracht werden. Der Rotor wird mit einem Gehäuse verbunden, welches ein Untersetzungsgetriebe aufweist. Über eine Antriebswelle wird der Rotor in eine Rotationsbewegung versetzt. Die Antriebswelle wird über einen externen Motor angetrieben, wobei Standardmotoren verwendet werden, die eine Geschwindigkeit von 40 000 Umdrehungen pro Minute aufweisen. Diese Drehzahl des Motors muss jedoch reduziert werden, um eine Überhitzung des Werkzeuges, also des Rotors zu vermeiden. Hierfür ist in der FR 2 765 471 A1 das Untersetzungsgetriebe vorgesehen, welches die Umdrehungen des Motors auf 10 000 Umdrehungen pro Minute für den Rotor reduziert. Dies ist bautechnisch sehr aufwendig und erhöht die Reparaturanfälligkeit des Handgerätes, da am Übersetzungsgetriebe Schäden auftreten können. Zudem ist es notwendige eine externe Motoreinheit mitzuführen, mit der das Handgerät im Betrieb verbunden werden muss. Dies schränkt die Flexibilität des Handgerätes in der Anwendung stark ein.

Darüber hinaus können zwar im Handgerät der FR 2 765 471 A1 die Klingen des Rotors ausgetauscht werden, jedoch müssen hierfür mehrere Schraubverbindungen gelöst werden. Dies ist zeitaufwendig und der direkte Kontakt mit den Klingen birgt zudem ein erhöhtes Verletzungsrisiko für den Nutzer. Dies ist auch unter einem hygienischen Aspekt bedenklich, da die Klingen nach der Nutzung mit den entfernten Hornhautresten kontaminiert sind.

Um ein hygienisch einwandfreies arbeiten zu ermöglichen, muss zudem beim Austausch der Klingen auch der Rotor gereinigt werden, da nicht nur die Klingen, sondern auch der Rotor mit der abgehobelten Hornhaut in Kontakt kommt. Wird das Gerät für verschiedene Patienten benutzt, sind die Hygieneanforderungen sehr hoch.

Ausgehend vom Stand der Technik ist es daher die Aufgabe der Erfindung ein Handgerät zur Verfügung zu stellen, welches die genannten Nachteile behebt.

Hierfür stellt die Erfindung ein Handgerät gemäß Anspruch 1 und einen Rotor gemäß Anspruch 6 zur Verfügung.

### Detaillierte Beschreibung

Erfindungsgemäß wird ein Handgerät zum Abrieb von Hornhaut aufweisend ein Gehäuse, einen Rotor und einen Stator zur Verfügung gestellt.

Im Gehäuse des Handgerätes ist ein Motor mit einer Antriebswelle angeordnet. Bevorzugt ragt die Antriebswelle teilweise aus dem Gehäuse hinaus, so dass ein Rotor auf die Antriebswelle aufgesteckt werden kann.

Das Gehäuse ist bevorzugt ergonomisch geformt, so dass das Handgerät gut in der Hand liegt und ein sicheres Arbeiten gewährleistet ist.

In einer Ausführungsform weist das Gehäuse mindestens ein Anzeigeelement auf. Das Anzeigeelement ist bevorzugt am Gehäuse derart angeordnet, dass es von einem Nutzer gut einsehbar ist.

Weiterhin ist am Gehäuse ein Schalter zum Ein- und Ausschalten sowie zur Steuerung des Handgerätes vorgesehen. In einer bevorzugten Ausführungsform ist dieser Schalter im Bereich des mindestens einen Anzeigeelementes angeordnet.

Wir das Handgerät eingeschaltet, kann dies beispielsweise durch das Aufleuchten einer Signallampe im Anzeigeelement dargestellt werden. In einer Ausführungsform kann der Motor im Handgerät mit verschiedenen Geschwindigkeitsstufen betrieben werden. In diesem Fall kann die jeweilige Stufe, in der der Motor läuft, durch eine Signallampe oder eine Anzeige im Anzeigeelement dargestellt werden.

Der Motor im Gehäuse treibt die Antriebswelle bevorzugt mit maximal 10 000 Umdrehungen pro Minute an. In einer Ausführungsform der vorliegenden Erfindung kann der Motor die Antriebswelle mit unterschiedlichen Drehzahlen antreiben, beispielsweise mit zwei, drei oder vier verschiedenen Drehzahlen.

In einer bevorzugten Ausführungsform kann der Motor die Antriebswelle mit genau einer Drehzahl, beispielsweise mit 10 000 Umdrehungen pro Minute antreiben.

In einer weiteren bevorzugten Ausführungsform kann der Motor die Antriebswelle mit zwei verschiedenen Drehzahlen antreiben. Eine Drehzahl davon kann beispielsweise 10 000 Umdrehungen pro Minute betragen.

In einer Ausführungsform erfolgt die Stromversorgung des Motors über ein Kabel durch eine externe Stromquelle. In dieser Ausführungsform ist im Gehäuse ein Durchlass für eine Stromkabel vom Motor zur Stromquelle vorgesehen.

In einer weiteren Ausführungsform weist das Gehäuse weiterhin Akkus zur Stromversorgung des Motors auf. Bevorzugt können die Akkus, während diese im Gehäuse verbleiben, durch entsprechende Vorrichtungen in einer Ladeschale geladen werden. Analog einer elektrischen Zahnbürste die bei Nichtbenutzung in einer Ladeschale aufbewahrt wird. Diese Ausgestaltung hat den Vorteil, dass bei der Nutzung des Handgerätes größtmögliche Flexibilität vorhanden ist, da der Bewegungsraum des Handgerätes nicht durch ein Stromkabel eingeschränkt wird.

Weiterhin weist das Handgerät einen Rotor mit zwei Klingen auf. Der Rotor wird auf die Antriebswelle des Motors aufgesteckt und kann dadurch bei Betrieb des Handgerätes in Rotation versetzt werden. Erfindungsgemäß kann der gesamte Rotor dadurch einfach, sicher und schnell ausgewechselt werden. Vorteilhafterweise müssen dabei nicht die Klingen berührt werden, womit die Verletzungsgefahr geringgehalten wird. Zudem werden durch das Austauschen des gesamten Rotors die hygienischen Bedingungen verbessert, da eine Reinigung einzelner Komponenten entfällt.

In einer Ausführungsform weist die Antriebswelle des Handgerätes einen polygonalen Querschnitt auf. Der Rotor weist bevorzugt eine axiale Bohrung auf, in die die Antriebswelle aufgenommen werden kann. Der Querschnitt der axialen Bohrung und der Antriebswelle sind daher bevorzugt identisch. Durch den polygonalen Querschnitt wird ein Verrutschen der Antriebswelle in der axialen Bohrung des Rotors bei der Drehung verhindert und der Rotor kann unproblematisch durch die Antriebwelle in Rotation versetzt werden. In einer besonders bevorzugten Ausführungsform weist die Antriebswelle einen hexagonalen Querschnitt auf.

Erfindungsgemäß wird der Rotor auf die Antriebswelle des Gehäuses gesteckt und anschließend der Stator über den Rotor. Der Stator wird dann am Gehäuse befestigt. Der Stator erfüllt dabei die Funktion einer Kappe für den Rotor, der sich im laufenden Betrieb innerhalb des Stators frei dreht. Der Stator kann beispielsweise durch ein Drehgewinde oder einen Klickmechanismus am Gehäuse befestigt werden. Die Verbindung zwischen Stator und Gehäuse ist dabei in jedem Fall eine einfach lösbare Verbindung, um ein Auswechseln des Rotors bei Bedarf einfach und bevorzugt ohne Einsatz eines Werkzeuges zu ermöglichen.

Der Stator hat die Form einer Hülse, deren Innenbohrung im Wesentlichen dem Durchmesser des Rotors entspricht und diesen aufnehmen kann. Der Stator weist ein Fenster auf, dessen Länge im Wesentlichen der Länge der Klingen des Rotors entspricht. Das Fenster des Stators ist dabei so angeordnet, dass, wenn der Rotor in den Stator gesteckt ist, das Fenster des Stators auf der Höhe der Klingen des Rotors ist. Bevorzugt sind die Kanten des Fensters zum Inneren des Stators hin abgeschrägt.

Die Klingen des Rotors ragen einige Zehntelmillimeter aus dem Fenster des Stators hinaus. Durch die Drehung des Rotors ragen beide Klingen abwechselnd aus dem Fenster des Stators hinaus. Dieser Vorsprung von zum Beispiel 0,5 mm ist ausreichend, um einen guten Abrieb der Hornhautschichten zu bewirken, ohne Verletzungen zu verursachen.

Weiterhin stellt die Erfindung einen Rotor zum Einsatz in einem Handgerät zum Abrieb von Hornhaut zur Verfügung. Der Rotor weist einen zylinderförmigen Körper und zwei Klingen auf.

Der zylinderförmige Körper weist eine axiale Bohrung zur Aufnahme einer Antriebswelle auf. Erfindungsgemäß entspricht der Querschnitt der axialen Bohrung dem Querschnitt der Antriebswelle. In einer bevorzugten Ausführungsform weist die axiale Bohrung einen hexagonalen Querschnitt auf.

Weiterhin weist der zylinderförmige Körper zwei Aufnahmevorrichtungen auf, wobei jede Aufnahmevorrichtung eine Klinge aufnehmen kann. Die erste Aufnahmevorrichtung und die zweite Aufnahmevorrichtung sind diametral zueinander angeordnet, so dass auch die beiden Klingen diametral zueinander angeordnet sind.

In einer Ausführungsform weist die Aufnahmevorrichtungen zwei Klingenhaltestifte auf, auf die eine Klinge aufgesteckt werden kann. Die Klingenhaltestifte sind so lang, dass ein Anteil nach dem Aufstecken der Klinge über diese hinausragt. Dieser Anteil wird geschmolzen, um die Klinge zu fixieren. Die Klinge weist für das Aufstecken auf die Klingenhaltestifte zwei Durchgangsbohrungen auf, die so angeordnet sind, dass die Durchgangsbohrungen auf die Klingenhaltestifte passen.

In einer weiteren Ausführungsform weist die Aufnahmevorrichtung mindestens zwei Klammern auf, an denen eine Klinge befestigt werden kann. Bevorzugt weist die Aufnahmevorrichtung mehr als zwei Klammern, nämlich 3, 4, 5 oder 6 Klammern auf, an denen die Klinge befestigt werden kann.

In einer Ausführungsform ist der Rotor sterilisierbar.

In einer bevorzugten Ausführungsform wird der erfindungsgemäße Rotor als Rotor in dem erfindungsgemäßen Handgerät eingesetzt. In diesem Fall gelten alle Ausführungen, die zu dem erfindungsgemäßen Handgerät gemacht wurden in gleicher Weise für den erfindungsgemäßen Rotor und umgekehrt. Gegenstand der Erfindung ist daher ebenfalls ein Handgerät zum Abrieb von Hornhaut aufweisend ein Gehäuse, einen Rotor und einen Stator, dadurch gekennzeichnet, dass
- im Gehäuse ein Motor mit einer Antriebswelle angeordnet ist;
- der Rotor auswechselbar ist;
- der Rotor auf die Antriebswelle des Motors aufsteckbar ist, der Stator auf den Rotor aufsteckbar ist und der Stator am Gehäuse befestigbar ist; und
- der Rotor einen zylinderförmigen Körper und zwei Klingen aufweist, wobei
   oder zylinderförmige Köper eine axiale Bohrung zur Aufnahme einer Antriebswelle aufweist;
   oder zylinderförmige Körper zwei Aufnahmevorrichtungen aufweist, wobei jede Aufnahmevorrichtung eine Klinge aufnehmen kann.

Im Folgenden wird die Erfindung anhand von 5 Figuren näher beschrieben.
- Figur 1: (A) und (B) stellen eine Ausführungsform des erfindungsgemäßen Handgerätes dar;
- Figur 2: (A) und (B) stellen eine weitere Ausführungsform des erfindungsgemäßen Handgerätes dar;
- Figur 3: stellt einen Stator, einen Rotor und einen Teil des Gehäuses dar;
- Figur 4: (A) stellt einen Stator, eine Ausführungsform eines Rotors und einen Teil des Gehäuses dar, (B) stellt die Ausführungsform des Rotors mit eingesetzten Klingen dar;
- Figur 5: (A) stellt einen Stator, eine Ausführungsform eines Rotors und einen Teil des Gehäuses dar, (B) stellt die Ausführungsform des Rotors dar.

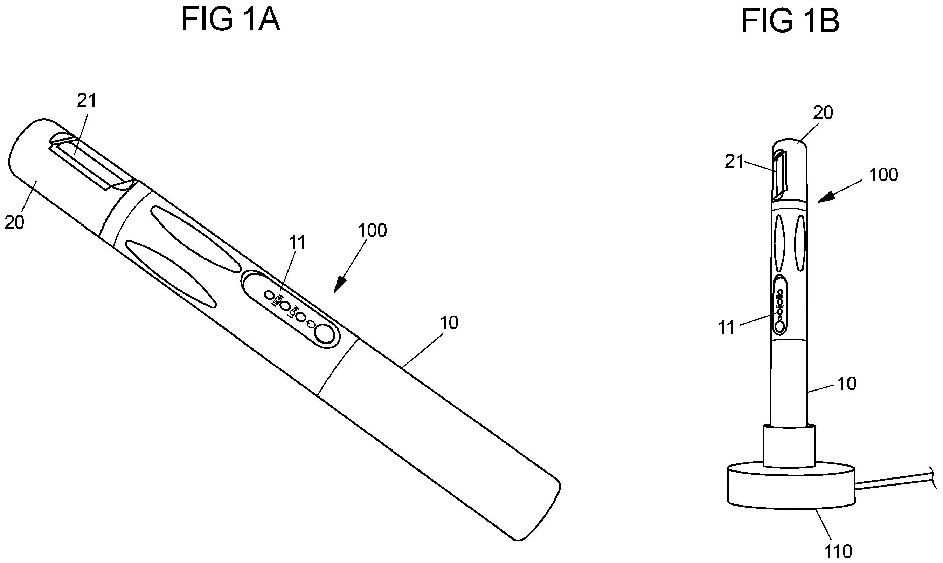

**Figur 1A** stellt ein erfindungsgemäßes Handgerät 100 mit einem Gehäuse 10 und einem Stator 20 dar. Der Stator 20 ist am Gehäuse 10 befestigt, daher ist der Rotor 30 nicht zu erkennen. Das Gehäuse 10 weist ein Anzeigeelement 11 auf, welches den An/Ausschalter und drei Signallampen aufweist. Am Stator 20 ist ein Fenster 21 angeordnet, durch welches die Klingen des Rotors 30 hinausragen können. In dieser Ausführungsform sind im Gehäuse Akkus angeordnet. **Figur 1B** stellt das erfindungsgemäße Handgerät in einer Ladeschale 110 dar. Die Ladeschale 110 wird über eine externe Stromquelle mit Strom versorgt und lädt die Akkus des Handgerätes 100 während dieses nicht genutzt wird. Die Akkus verbleiben hierfür im Gehäuse 10.

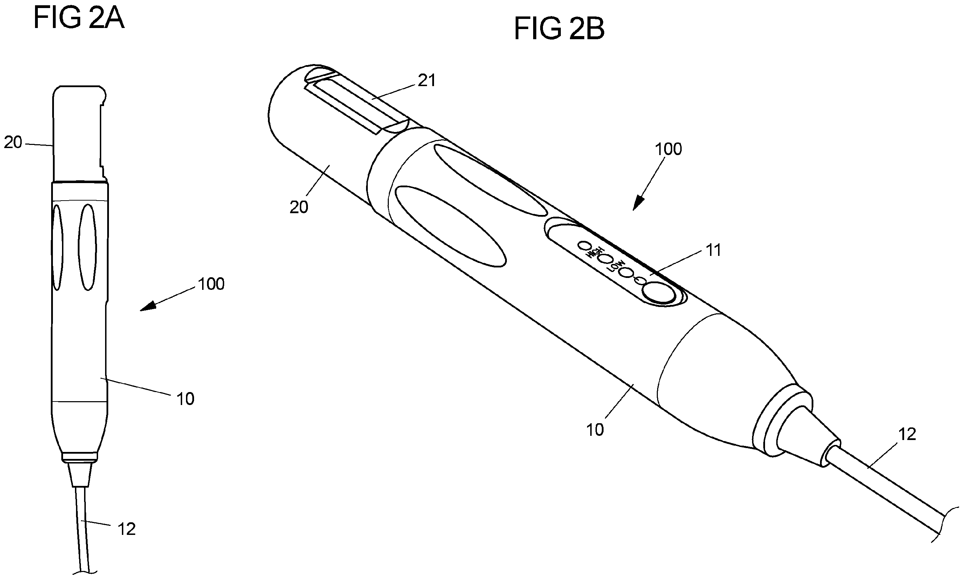

**Figur 2A** stellt ein erfindungsgemäßes Handgerät 100 mit einem Gehäuse 10 und einem Stator 20 dar. Der Stator 20 ist am Gehäuse 10 befestigt, daher ist der Rotor 30 nicht zu erkennen. Das Handgerät 100 wird in dieser Ausführungsform über ein Kabel 12 durch eine externe Stromquelle mit Energie versorgt. **Figur 2B** zeigt das Handgerät 100 aus einer weiteren Perspektive. Es ist zu erkennen, dass das Gehäuse 10 ein Anzeigeelement 11 aufweist, welches den An/Ausschalter und drei Signallampen aufweist. Am Stator 20 ist ein Fenster 21 angeordnet, durch welches die Klingen des Rotors 30 hinausragen können.

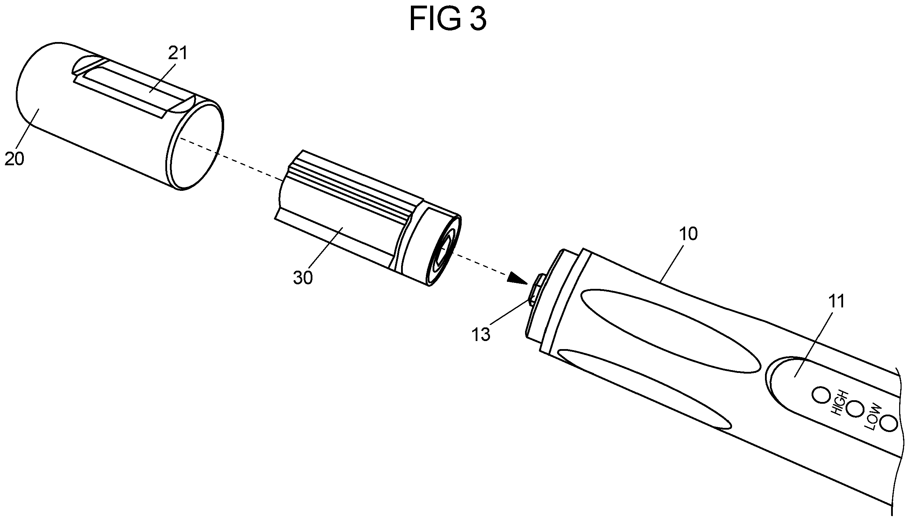

**Figur 3** stellt das Zusammensetzten von Stator 20, Rotor 30 und Gehäuse 10 dar. Der Rotor 30 wird auf die Antriebswelle 13 des Gehäuses 10 gesteckt und der Stator 20 auf den Rotor 30. Anschließend wird der Stator 20 mit dem Gehäuse 10 verbunden.

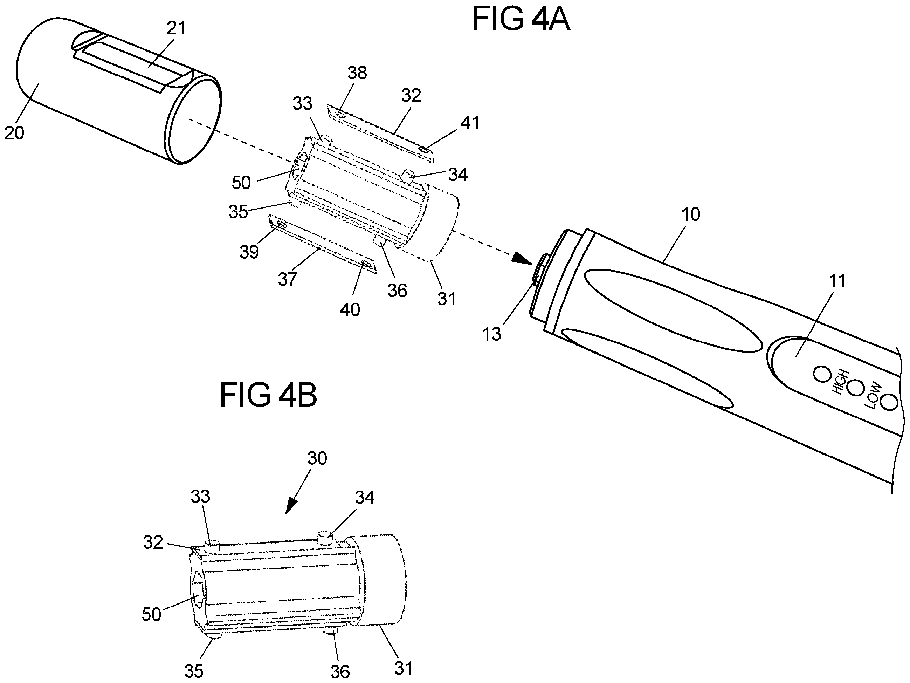

**Figur 4A** stellt einen Stator 20, einen Rotor 30 und ein Gehäuse 10 dar. Die Figur stellt eine Ausführungsform des Rotors 30 dar, bei der die zwei Klingen 32, 37 auf die Klingenhaltestifte 33, 34, 35, 36 aufgesteckt werden. Die Klingenhaltestifte 33-36 sind so lang, dass ein Anteil nach dem Aufstecken der Klinge 32, 37 über diese hinausragt. Dieser Anteil wird geschmolzen, um die Klinge 32, 37 zu fixieren. Die Klinge 32 weist für das Aufstecken auf die Klingenhaltestifte 33, 34 zwei Durchgangsbohrungen 38, 41 auf, die so angeordnet sind, dass die Durchgangsbohrungen 38, 41 auf die Klingenhaltestifte 33, 34 passen. Analog gilt dies für die Klinge 37 mit den Durchgangsbohrungen 39, 40, die auf die Klingenhaltestifte 35, 36 passen.

Der Rotor 30 weist einen zylindrischen Körper 31 mit einer axialen Bohrung 50 auf. Die axiale Bohrung 50 hat in dieser Ausführungsform den gleichen hexagonalen Querschnitt wie die Antriebswelle 13 des Gehäuses. Die Klingenhaltestift 33-36 sind Teil des zylinderförmigen Körpers.

**Figur 4B** stellt den Rotor 30 noch einmal mit montierten Klingen 32, 37 dar.

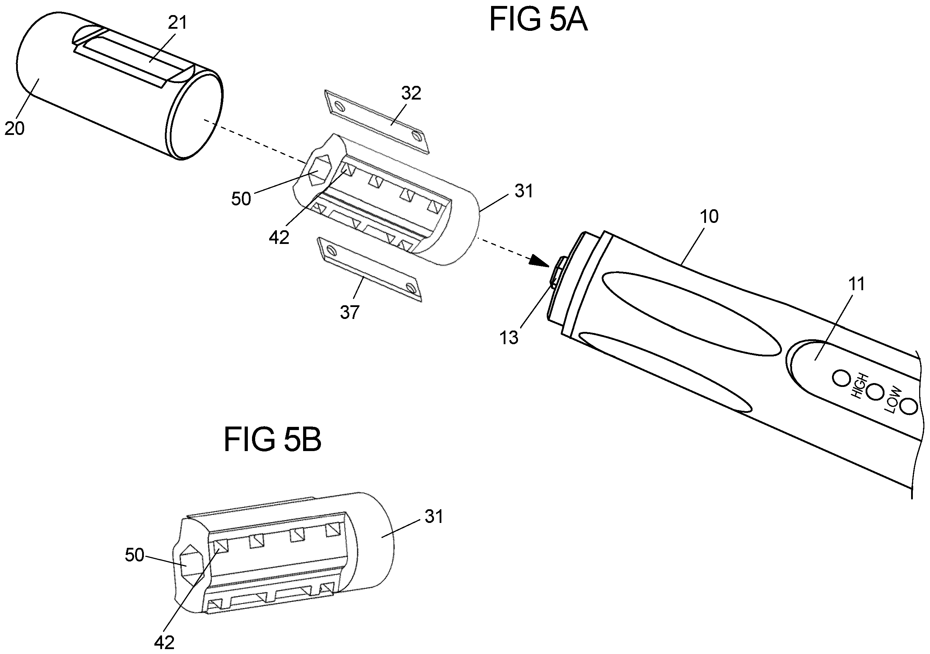

**Figur 5A** stellt einen Stator 20, einen Rotor 30 und ein Gehäuse 10 dar. Die Figur stellt eine Ausführungsform des Rotors 30 dar, bei der die zwei Klingen 32, 37 über Klammern 42 am zylindrischen Körper 31 des Rotors fixiert werden. **Figur 5B** stellt den Rotor 30 noch einmal in einer weiteren Ansicht dar.

### Bezugszeichen

- 10: Gehäuse
- 11: Anzeigeelement
- 12: Kabel
- 13: Antriebswelle
- 20: Stator
- 21: Fenster
- 30: Rotor
- 31: zylindrischer Körper
- 32, 37: Klinge
- 33-36: Klingenhaltestift
- 38-41: Durchgangsbohrung
- 42: Klammern
- 50: axiale Bohrung
- 100: Handgerät
- 110: Ladeschale

## Patentansprüche

1. Handgerät zum Abrieb von Hornhaut aufweisend ein Gehäuse (10), einen Rotor (30) und einen Stator (20), **dadurch gekennzeichnet, dass**
• im Gehäuse (10) ein Motor mit einer Antriebswelle (13) angeordnet ist;
• der Rotor (30) zwei Klingen (32, 37) aufweist und auswechselbar ist;
• der Rotor (30) auf die Antriebswelle (13) des Motors aufsteckbar ist, der Stator (20) auf den Rotor (30) aufsteckbar ist und der Stator (20) am Gehäuse (10) befestigbar ist.

2. Handgerät gemäß Anspruch 1, **dadurch gekennzeichnet, dass** das Gehäuse (10) mindestens ein Anzeigeelement (11) aufweist.

3. Handgerät gemäß einem der vorangegangenen Ansprüche, **dadurch gekennzeichnet, dass** die Stromversorgung des Motors über ein Kabel durch eine externe Stromquelle erfolgt.

4. Handgerät gemäß einem der vorangegangenen Ansprüche, **dadurch gekennzeichnet, dass** das Gehäuse (10) weiterhin Akkus zur Stromversorgung des Motors aufweist.

5. Handgerät gemäß einem der vorangegangenen Ansprüche, **dadurch gekennzeichnet, dass** die Antriebswelle (13) einen polygonalen Querschnitt, bevorzugt einen hexagonalen Querschnitt aufweist.

6. Rotor (30) zum Einsatz in einem Handgerät zum Abrieb von Hornhaut, aufweisend einen zylinderförmigen Körper und zwei Klingen (32, 37) **dadurch gekennzeichnet, dass**
• der zylinderförmige Köper eine axiale Bohrung zur Aufnahme einer Antriebswelle (13) aufweist;
• der zylinderförmige Körper zwei Aufnahmevorrichtungen aufweist, wobei jede Aufnahmevorrichtung eine Klinge aufnehmen kann.

7. Rotor (30) gemäß Anspruch 6, **dadurch gekennzeichnet, dass** der Rotor (30) sterilisierbar ist.

8. Rotor (30) gemäß einem der Ansprüche 6 oder 7 **dadurch gekennzeichnet, dass** eine Aufnahmevorrichtungen zwei Klingenhaltestifte (33) aufweist, auf die eine Klinge (32) aufgesteckt werden kann und der Anteil der Klingenhaltestifte (33) der über die Klinge (32) hinausragt geschmolzen wird, um die Klinge (32) zu fixieren.

9. Rotor (30) gemäß einem der Ansprüche 6 oder 7 **dadurch gekennzeichnet, dass** eine Aufnahmevorrichtung mindestens zwei Klammern (42) aufweist, an denen eine Klinge (32) befestigt werden kann.

10. Verwendung des Rotors (30) gemäß einem der Ansprüche 6 bis 9 in einem Handgerät gemäß einem der Ansprüche 1 bis 5.
